# NEUE EUROPÄISCHE PATENTSCHRIFT

(11) **EP 0 848 592 B2**
(45) Veröffentlichungstag und Bekanntmachung des Hinweises auf die Entscheidung über den Einspruch: **09.03.2005**
(45) Hinweis auf die Patenterteilung: 02.11.2000
(21) Anmeldenummer: 96930072.2
(22) Anmeldetag: 22.08.1996
(51) Int. Cl.: A23K 1/18, A23K 1/16, A61K 31/715

(54) **ANTISTRESSMITTEL FÜR WASSERTIERE**
ANTISTRESS AGENTS FOR AQUATIC ANIMALS
AGENTS ANTISTRESS POUR ANIMAUX AQUATIQUES

(30) Priorität: 05.09.1995 DE 19532682
(43) Veröffentlichungstag der Anmeldung: 24.06.1998
(73) Patentinhaber: Tetra GmbH, 49324 Melle (DE)
(72) Erfinder: KÜRZINGER, Hubert, D-49324 Melle (DE)
(74) Vertreter: Miller, Andreas, Dr.
(86) Internationale Anmeldenummer: PCT/EP1996/003689
(87) Internationale Veröffentlichungsnummer: WO 1997/008960

(56) Entgegenhaltungen:
- EP-A- 0 466 037
- WO-A-95/04467
- DE-A- 4 335 454
- FR-A- 2 674 755
- DATABASE WPI Week 9046 Derwent Publications Ltd., London, GB; AN 90-345661 XP002020862 & JP,A,02 250 832 (IKEDA TOKA KOGYO KK) , 8.Oktober 1990
- DATABASE WPI Week 9408 Derwent Publications Ltd., London, GB; AN 94-063313 XP002020863 & SU,A,1 788 893 (UNIV. ZAPORO) , 15.Januar 1993
- DATABASE WPI Week 8347 Derwent Publications Ltd., London, GB; AN 83-823682 XP002020864 & JP,A,58 175 451 (SEISAN KAIHATSU KAGAKU) , 14.Oktober 1983
- DATABASE WPI Week 8209 Derwent Publications Ltd., London, GB; AN 82-16701E XP002020865 & JP,A,57 012 937 (ORIENTAL YEAST KK) , 22.Januar 1982
- DATABASE WPI Week 8728 Derwent Publications Ltd., London, GB; AN 87-197197 XP002020866 & SU,A,1 271 520 (KIEV PHYSICAL MED.) , 23.November 1986
- DATABASE WPI Week 9412 Derwent Publications Ltd., London, GB; AN 94-97780 XP002020867 & JP,A,06 048 949 (TAITO KK) , 22.Februar 1994
- DATABASE WPI Week 9443 Derwent Publications Ltd., London, GB; AN 94-347060 XP002020868 & JP,A,06 271 470 (ASAHI KASEI KOGYO KK) , 27.September 1994 in der Anmeldung erwähnt

## Beschreibung

Die Erfindung betrifft Antistreßmittel zur Verbesserung der Widerstandsfähigkeit von Wassertieren, insbesondere Fische, Shrimps und Invertebraten im Süß- und Seewasser, bei Belastungen aller Art, einsetzbar auch als Antistreßmittel für Warm- und Kaltwasser-Zierfische enthaltend ein Vitamin oder eine Kombination von Vitaminen in Megadosen und einem oder mehreren Immunstimulatoren, in Form eines Futtermittels in Form von Gemischen, Flocken, Extrudaten, Pellets oder Tabletten im trockenen, feuchten oder halbfeuchten Zustand.

Bekannt ist die Verwendung von Immunstimulatoren (z.B. Glucan) zum Schutz vor bakteriellen und viralen Infektionskrankheiten bei Shrimps aus AU 92 10 574. Die Applikation erfolgt über das Futter in einer Dosierung von 0,001 - 10 %. Wie in JP 22 18 615 wird auch in EP-A-0 466 037 die Erhöhung der Widerstandsfähigkeit von Fischen und Shrimps gegen Krankheitserreger beschrieben. Das Trockenfutter enthält 5 - 100 mg Glucan pro kg Futter. In der EP-A-0 384 323 wird der synergistische Effekt einer Vaccinierung gegen Aeromonas bei Fischen durch gleichzeitige Verabreichung von 15 - 20 mg Glucan über das Futter pro kg Körpergewicht beschrieben. Weiterhin bekannt ist aus der EP-A-0 559 450 der Einsatz von Glucan als Bindemittel im Fischfutter.

In der JP 2250832 ist weiterhin ein Immunpotentiator für Fische beschrieben, der 20 mg Glycyrrhizin pro kg enthält und dem 10 mg Vitamin E als Antioxidationsmittel beigegeben sind.

Die Vitamingehalte in den üblicherweise verwendeten natürlichen Rohstoffen, die zu Mischfutter für aquatische Tiere verarbeitet werden, sind meist nicht ausreichend, um Mangelsymptomen vorzubeugen. Denn mit zu den häufigsten ernährungsbedingten Krankheiten zählen Vitaminmangelerscheinungen. Aus diesem Grund werden üblicherweise Futtermitteln für Fische und Shrimps Vitamine in bedarfsdeckenden Mengen zugesetzt. Handelsübliche Dosierungen sind z. B. in NRC, Nutrient Requirements of Warmwater Fishes and Shellfishes, 1983 beschrieben. Die empfohlenen handelsüblichen Bedarfswerte an Vitaminen für Fische (z.B. Cyprinus carpio L.) betragen pro kg Futter 10000 IE Vitamin A, 500 - 1000 IE Vitamin D, 30 mg Vitamin E. 1 mg Vitamin B₁, 9 mg Vitamin B₂, 3 mg Vitamin B₆, 60 mg Vitamin C, 10 - 20 mg Pantothensäure, 14 mg Nicotinsäure. Bedarfsangaben für Vitamin B₁₂, Vitamin K, Inosit, Cholin, Folsäure und Vitamin C-Äquivalent aus langzeitstabilem Vitamin C-Phosphat sind nicht dokumentiert.

Die Auswirkungen von Überdosierungen von Vitaminen wurde durch den Einsatz von Megadosen an Vitaminen in der wissenschaftlichen Literatur beschrieben (z.B. Steffens: Grundlagen der Fischernährung, 1985). Bei den Untersuchungen wurde vorrangig darauf geachtet, ob Hypervitaminosen wie erhöhte Mortalität, reduziertes Wachstum, ungünstige, Futterverwertung, starke Defekte bedingt durch Überdosierungen an Vitaminen auftraten, z.B. durch die Verfütterung von 3,75 x 10⁶ IE Vitamin D₃/kg Futter; 2 x 10⁶ IE Vitamin A/kg Futter; 5 g Vitamin E/kg Futter; 10 g Nicotinsäure/kg Futter. Die überprüften Megadosen zeigten aber keine negativen Effekte.

Überraschenderweise wurde nun gefunden, daß Antistreßmittel, insbesondere Futtermittel, enthaltend eine Überdosierung von einem oder mehreren Vitaminen in Kombination mit einem oder mehreren Immunstimulatoren hervorragend geeignet sind, die Widerstandsfähigkeit von Wassertieren bei Belastungen aller Art, insbesondere aber durch Streß, zu erhöhen.

Streßsituationen treten bei Wassertieren nahezu permanent auf und führen zu einer starken Belastung der Tiere. Beispiele für Streßsituationen sind Hunger, hohe Besatzdichte, Wasserwechsel, Veränderungen der Wasserparameter, Revierkämpfe. Aggressionsverhalten, Handling, medikamentöse Therapie, Transport, Krankheiten.

Gegenstand der Erfindung sind daher Antistreßmittel für Wassertiere, insbesondere Fische, Shrimps und Invertebraten in Süß- und Seewasser, gemäß Anspruch 1.

Der Immunstimulator ist im Antistreßmittel in einer Menge von 0,0001 - 10 Gew.-%, bevorzugt 0,1 Gew.-% vorhanden und ist ein β-Glucan.

Die Vitamine werden wie folgt dosiert (pro kg Futtermittel):

| | |
|---|---|
| Vitamin A | 2,8 x 10⁵ IE |
| Vitamin D | 2,5 x 10³ IE |
| Vitamin E | 1,9 g |
| Vitamin B₁ | 330 mg |
| Vitamin B₂ | 950 mg |
| Vitamin B₆ | 190 mg |
| Vitamin B₁₂ | 820 µg |
| Vitamin C | 6,35 g |
| Vitamin K | 20 mg - 5 g |
| Pantothensäure | 940 mg |
| Nicotinsäure | 4,7 g |
| Inosit | 7.3 g |
| Cholin | 200 mg - 50 g |
| Folsäure | 96 mg |
| Vitamin C-Äquivalent | 0.1 mg - 5 g |

Besonders bevorzugt ist ein Mittel, enthaltend pro kg Futter:

| | |
|---|---|
| Vitamin A | 2,8 x 10⁵ IE |
| Vitamin D | 2,5 x 10³ IE |
| Vitamin E | 1,9 g |
| Vitamin B₁ | 330 mg |
| Vitamin B₂ | 950 mg |
| Vitamin B₆ | 190 mg |
| Vitamin B₁₂ | 820 µg |
| Vitamin C | 6,35 g |
| Vitamin K | 96 mg |
| Pantothensäure | 940 mg |
| Nicotinsäure | 4,7 g |
| Inosit | 7,3 g |
| Cholin | 1,13 g |
| Folsäure | 96 mg |
| Vitamin C-Äquivalent | 4,9 g |
| β-Glucan | 1 g |

Das neuartige Antistreßmittel für Wassertiere wurde über das Futter an territorialen, revierbildenden Zierfischen (Cichlasoma nicaraguense) bezüglich Streßverhalten im Vergleich zur Kontrolle (ohne Megadosen an Vitaminen und ohne Immunstimulatoren) mit je drei Parallelen getestet. Neben dem permanent vorhandenen sozialen Streß wurde zusätzlich täglich in jedem Aquarium Streß induziert, indem mit einem Fischfangnetz im Becken mehrfach gerührt wurde.

Die erhöhten Verluste von 19 % in der Kontrollgruppe sind auf den verstärkten Streß durch das Aggressionsverhalten und vor allem auf den verstärkten Streß durch die Behandlung mit dem Fangnetz zurückzuführen. Hiervon waren alle Tiergrößen betroffen. Die mit dem Versuchsfutter ernährten Barsche zeigten aufgrund des Futters, das Megadosen an Vitaminen und einen Immunstimulator enthielt, fast keine Ausfälle (1 % Mortalität).

Das erfindungsgemäße Antistreßmittel kann über das Futter als Flocken, Extrudate, Pellets und Tabletten im trockenen, feuchten oder halbfeuchten Zustand appliziert werden. Die Anwendung kann prophylaktisch oder auch bei akuten Belastungen der Wassertiere erfolgen.

Die Applikation des Antistreßmittels erfolgt in einer Kombination aus Megadosen an einem oder mehreren Vitaminen und einem oder mehreren Immunstimulatoren.

## Patentansprüche

1. Futtermittel für Wassertiere in Form von Gemischen, Flocken, Extrudaten, Pellets oder Tabletten im trockenen, feuchten oder halbfeuchten Zustand enthaltend
a) β-Glucan in einer Menge von 0,0001 bis 10 Gew.-%, bevorzugt 0,1 Gew.-%, sowie
b) pro kg Futtermittel eine Überdosierung von mindestens einem Vitamin ausgewählt aus 280.000 IE Vitamin A, 2500 IE Vitamin D, 1900 mg Vitamin E, 330 mg Vitamin B₁, 950 mg Vitamin B₂, 190 mg Vitamin B₆, 820 µg Vitamin B₁₂, 6350 mg Vitamin C, 0,1 - 5000 mg Vitamin C-Äquivalent, 20 - 5000 mg Vitamin K, 940 mg Pantothensäure, 4700 mg Nicotinsäure, 7300 mg Inosit, 200 - 50.000 mg Cholin und 96 mg Folsäure zur Erhöhung der Widerstandfähigkeit von Wassertieren bei Belastungen aller Art, insbesondere Stress.

2. Futtermittel gemäß Anspruch 1, **dadurch gekennzeichnet, dass** es pro kg Futtermittel
a) 1 g β-Glucan sowie
b) 280.000 IE Vitamin A, 2500 IE Vitamin D, 1900 mg Vitamin E, 330 mg Vitamin B₁, 950 mg Vitamin B₂, 190 mg Vitamin B₆, 820 µg Vitamin B₁₂,6350 mg Vitamin C, 4900 mg Vitamin C-Äquivalent, 96 mg Vitamin K, 940 mg Pantothensäure, 4700 mg Nicotinsäure, 7300 mg Inosit, 1130 mg Cholin und 96 mg Folsäure enthält.

3. Verwendung von β-Glucan in einer Menge von 0,0001 bis 10 Gew.-%, bevorzugt 0,1 Gew.-%, sowie pro kg Futtermittel eine Überdosierung von mindestens einem Vitamin ausgewählt aus 280.000 IE Vitamin A, 2500 IE Vitamin D, 1900 mg Vitamin E, 330 mg Vitamin B₁, 950 mg Vitamin B₂, 190 mg Vitamin B₆, 820 µg Vitamin B₁₂,-6350 mg Vitamin C, 0,1 - 5000 mg Vitamin C-Äquivalent, 20 - 5000 mg Vitamin K, 940 mg Pantothensäure, 4700 mg Nicotinsäure, 7300 mg Inosit, 200 - 50.000 mg Cholin und 96 mg Folsäure bei der Herstellung von Futtermitteln für Wassertiere zur Erhöhung der Widerstandfähigkeit und Verhinderung von Verlusten bei der Haltung.

## Claims

1. Feed for aquatic animals in the form of mixtures, flakes, extrudates, pellets or tablets in dry, moist or semi-moist condition containing
a) β-glucane in an amount of 0,0001 to 10 weight-%, preferred 0,1 weight-% and
b) per kg feed an overdosage of at least a vitamin selected from 280.000 IU vitamin A, 2500 IU vitamin D, 1900 IU vitamin E, 330 mg vitamin B₁, 950 mg vitamin B₂, 190 mg vitamin B₆, 820 µg vitamin B₁₂, 6350 mg vitamin C, 0,1 - 5000 mg vitamin C equivalent, 20 - 5000 mg vitamin K, 940 mg pantothenic acid, 4700 mg nicotinic acid, 7.300 mg inositol, 200- 50.000 mg choline, 96 mg folic acid
for the improvement the resistance of aquatic animals in case of strains of all kinds but especially of stress

2. Feed according to Claim 1, wherein it contains per kg
a) 1g β-glucane and
b) 280.000 IU vitamin A, 2500 IU vitamin D, 1900 IU vitamin E, 330 mg vitamin B₁, 950 mg vitamin B₂, 190 mg vitamin B₆, 820 µg vitamin B₁₂, 6350 mg vitamin C, 4900 mg vitamin C equivalent, 96 mg vitamin K, 940 mg pantothenic acid, 4700 mg nicotinic acid, 7300 mg inositol, 1130 mg choline and 96 mg folic acid.

3. Use of β-glucane in an amount of 0,0001 weight-% to 10 weight-%, preferred 0,1 weight-%, preferred 0,1 weight-%, and per kg of feed an overdosage of at least one vitamin selected from 280.000 lU vitamin A, 2500 IU vitamin D, 1900 IU vitamin E, 330 mg vitamin B₁, 950 mg vitamin B₂, 190 mg vitamin B₆, 820 µg vitamin B₁₂, 6350 mg vitamin C, 0,1 - 5.000 mg vitamin C equivalent, 20 - 5000 mg vitamin K, 940 mg pantothenic acid, 4700 mg nicotinic acid, 7300 mg inositol, 200 - 50.000 mg choline and 96 mg folic acid, for the manufacturing of feed for aquatic animals for the improvement for the resistance and the prevention of losses in the keeping of said aquatic animals.

## Revendications

1. Aliment pour animaux aquatiques sous forme de mélanges, flocons, extrudats, pellets ou tablettes à l'état sec, humide ou semi-humide contenant
a) β-glucane en une quantité de 0,0001 à 10 % en poids, de préférence 0,1 % en poids et
b) par kg d'aliment un surdosage d'au moins une vitamine choisie parmi 280.000 UI de vitamine A, 2500 UI de vitamine D, 1900 UI de vitamine E, 330 mg de vitamine B₁, 950 mg de vitamine B₂, 190 mg de vitamine B₆, 820 µg de vitamine B₁₂, 6350 mg de vitamine C, 0,1 - 5000 mg d'équivalent de vitamine C, 20 - 5000 mg de vitamine K, 940 mg d'acide pantothénique, 4700 d'acide nicotinique, 7300 mg d'inosit, 200- 50.000 mg de choline et 96 mg d'acide folique,
pour augmenter la résistance d'animaux aquatiques aux contraintes diverses, mais en particulier au stress.

2. Aliment selon la revendication 1, **caractérisé en ce qu'**il contient par kg d'aliment
a) 1 g de β-glucane et
b) 280.000 UI de vitamine A, 2500 UI de vitamine D, 1900 UI de vitamine E, 330 mg de vitamine B₁, 950 mg de vitamine B₂, 190 mg de vitamine B₆, 820 µg de vitamine B₁₂, 6350 mg de vitamine C, 4900 mg d'équivalent de vitamine C, 96 mg de vitamine K, 940 mg d'acide pantothénique, 4700 mg d'acide nicotinique, 7300 mg d'inosit, 1130 mg de choline et 96 mg d'acide folique.

3. Utilisation de β-glucane en une quantité de 0,0001 % en poids à 10 % en poids, de préférence de 0,1 % en poids, et par kg d'aliment un surdosage au moins d'une vitamine choisie par 280.000 UI de vitamine A, 2.500 UI de vitamine D, 1900 UI de vitamine E, 330 mg de vitamine B₁, 950 mg de vitamine B₂, 190 mg de vitamine B₆, 820 µg de vitamine B₁₂, 6350 mg de vitamine C, 0,1 - 5.000 mg d'équivalent de vitamine C, 20 - 5.000 mg de vitamine K, 940 mg d'acide pantothénique, 4700 mg d'acide nicotinique, 7300 mg d'inosit, 200 - 50.000 mg de choline et 96 mg d'acide folique, lors de la fabrication d'aliments pour animaux aquatiques pour l'amélioration de la résistance et pour éviter les pertes lors de l'élevage.
